(19) Europäisches Patentamt
European Patent Office
Office européen des brevets

(11) EP 0 632 796 B1

(12) EUROPEAN PATENT SPECIFICATION

(45) Date of publication and mention of the grant of the patent:
**05.01.2000 Bulletin 2000/01**

(21) Application number: **93907445.6**

(22) Date of filing: **11.03.1993**

(51) Int Cl.[7]: **C07C 15/08**, C07C 6/12, B01J 29/04

(86) International application number:
**PCT/US93/02255**

(87) International publication number:
**WO 93/17987 (16.09.1993 Gazette 1993/22)**

# (54) TOLUENE DISPROPORTIONATION PROCESS

DISPROPORTIONIERUNGSVERFAHREN VON TOLUOL

PROCESSUS DE DISPROPORTIONNEMENT DU TOLUENE

(84) Designated Contracting States:
**BE DE FR GB IT NL**

(30) Priority: **12.03.1992 US 850105**
**12.03.1992 US 850104**
**02.09.1992 US 939752**

(43) Date of publication of application:
**11.01.1995 Bulletin 1995/02**

(73) Proprietor: **MOBIL OIL CORPORATION (a New York corporation)**
**Fairfax, Virginia 22037 (US)**

(72) Inventors:
- **CHANG, Clarence, Dayton**
  **Princeton, NJ 08540 (US)**
- **RODEWALD, Paul, Gerhard, Jr.**
  **Rocky Hill, NJ 08553 (US)**

(74) Representative: **Kador & Partner**
**Corneliusstrasse 15**
**80469 München (DE)**

(56) References cited:

| | |
|---|---|
| EP-A- 0 289 691 | WO-A-93/17788 |
| FR-A- 2 566 389 | US-A- 4 090 981 |
| US-A- 4 127 616 | US-A- 4 465 866 |
| US-A- 4 477 583 | US-A- 4 851 604 |
| US-A- 4 891 458 | US-A- 4 950 853 |
| US-A- 4 962 257 | US-A- 5 030 787 |
| US-A- 5 053 573 | |

- **CHEMICAL ABSTRACTS, vol. 102, no. 13, 1 April 1985, Columbus, Ohio, US; abstract no. 113006z, page 667 ; & JP-A-59 190 928 (NIPPON PETROCHEMICAL)**
- **Jour. Catalysis, 1991, HIBINO et al., "Shape Selectively over HZSM-5 Modified by Chemical Vapor Deposition of Silicon Oxide", pp. 551-558.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 632 796 B1

Printed by Jouve, 75001 PARIS (FR)

## Description

The present invention is directed to a toluene disproportionation process and, in particular, to a process for the selective disproportionation of toluene to para-xylene.

The term shape-selective catalysis describes unexpected catalytic selectivities in zeolites. The principles behind shape selective catalysis have been reviewed extensively, e.g. by N.Y. Chen, W.E. Garwood and F.G. Dwyer, "Shape Selective Catalysis in Industrial Applications," 36, Marcel Dekker, Inc. (1989). Within a zeolite pore, hydrocarbon conversion reactions such as paraffin isomerization, olefin skeletal or double bond isomerization, oligomerization and aromatic disproportionation, alkylation or transalkylation reactions are governed by constraints imposed by the channel size. Reactant selectivity occurs when a fraction of the feedstock is too large to enter the zeolite pores to react; while product selectivity occurs when some of the products cannot leave the zeolite channels. Product distributions can also be altered by transition state selectivity in which certain reactions cannot occur because the reaction transition state is too large to form within the zeolite pores or cages. Another type of selectivity results from configurational diffusion where the dimensions of the molecule approach that of the zeolite pore system. A small change in dimensions of the molecule or the zeolite pore can result in large diffusion changes leading to different product distributions. This type of shape selective catalysis is demonstrated, for example, in the selective disproportionation of toluene to p-xylene.

Para-xylene is a very valuable commercial product useful in the production of polyester fibers. The catalytic production of para-xylene has received much attention in the scientific community and various methods for increasing catalyst para-selectivity have been described.

The synthesis of para-xylene is typically performed by methylation of toluene in the presence of a suitable catalyst. Examples are the reaction of toluene with methanol as described by Chen et al., J. Amer. Chem. Sec. 1979, 101, 6783, and toluene disproportionation, as described by Pines in "The Chemistry of Catalytic Hydrocarbon Conversions", Academic Press, N.Y., 1981, p. 72. Such methods typically result in the production of a mixture including para-xylene, ortho-xylene, and meta-xylene. Depending upon the para-selectivity of the catalyst and the reaction conditions, different percentages of para-xylene are obtained. The yield, i.e., the amount of feedstock actually converted to xylene, is also affected by the catalyst and the reaction conditions.

The equilibrium reaction for the disproportionation of toluene to xylene and benzene proceeds as follows:

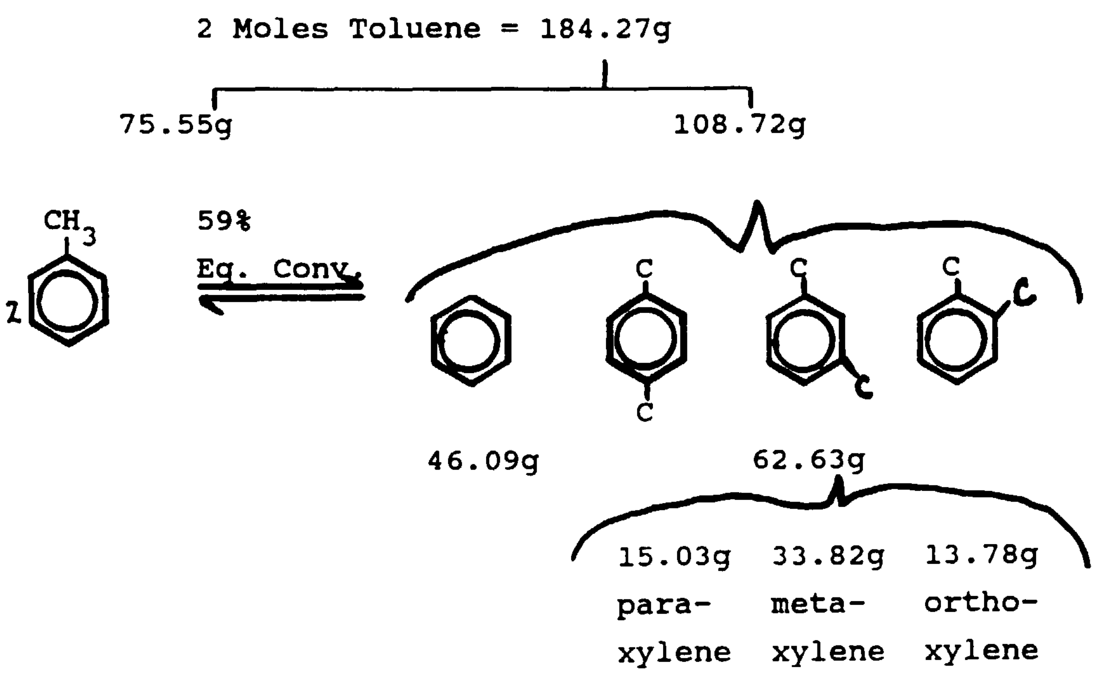


P-xylene=Selectivity x Conversion= $\frac{15.03}{108.72}$ x 0.59=8.2% Yield

p-Xylene purity=100 x $\frac{15.03}{62.63}$=24%

One known method for increasing para-selectivity of a zeolite catalyst is to modify the catalyst by treatment with a "selectivating agent". Modification methods have been suggested wherein the catalyst is modified by treatment prior to use to provide a silica coating. For example, U.S. Patents 4,477,583 and 4,127,616 disclose methods wherein

a catalyst is contacted at ambient conditions with a modifying compound such as phenylmethyl silicone in a hydrocarbon solvent or an aqueous emulsion, followed by calcination. Such modification procedures have been successful in obtaining para-selectivity up to about 90% but only at the expense of commercially unacceptable toluene conversions of about 10%, resulting in a yield of not greater than about 9%, i.e. 10% x 90%. Such processes also produce significant quantities of ortho-xylene and meta-xylene thereby necessitating expensive separation processes, such as fractional crystallization and adsorptive separation, in order to separate the para-xylene from the other isomers. The other xylene isomers are customarily recycled, thereby requiring xylene isomerization units for additional conversion of the recycled xylene isomers into an equilibrium xylene mixture comprising para-xylene.

Those skilled in the art appreciate that the expense of the separation process is proportional to the degree of separation required. Therefore, significant cost savings could be achieved by increasing selectivity to the para-isomer while maintaining commercially acceptable conversion levels.

An object of the present invention is, therefore, to provide a regioselective process for the production of para-xylene from toluene while maintaining commercially acceptable toluene conversion levels.

Accordingly, the invention resides in a toluene disproportionation process comprising contacting a feed stream comprising toluene with a molecular sieve catalyst to provide a single pass product comprising at least about 90% para-xylene and a toluene conversion of at least about 15%.

The molecular sieve catalyst used in the process of the present invention preferably has an initial constraint index of 1-30, preferably 1-20, more preferably 1-12, and preferably comprises an intermediate pore-size zeolite such as a ZSM-5, ZSM-11, ZSM-22, ZSM-23, or ZSM-35, most preferably ZSM-5. The catalyst preferably has an alpha value greater than 100, for example 150 - 2000, and a silica-alumina ratio less than 100, preferably 20 - 80. The Alpha Value of the catalyst may be increased by treating the catalyst with nitric acid or by mild steaming as discussed in U. S. Patent No. 4,326,994.

The Alpha Value is an approximate indication of the catalytic cracking activity of the catalyst compared to a standard catalyst and it gives the relative rate constant (rate of normal hexane conversion per volume of catalyst per unit time.) It is based on the activity of an amorphous silica-alumina cracking catalyst taken as an Alpha of 1 (Rate Constant = 0.016 $sec^{-1}$). The Alpha Test is described in U.S. Patent 3,354,078 and in The Journal of Catalysis, Vol. 4, pp. 522-529 (August 1965): Vol. 6, p. 278 (1966); and Vol. 61, p. 395 (1980). It is noted that intrinsic rate constants for many acid-catalyzed reactions are proportional to the Alpha Value for a particular crystalline silicate catalyst (see "The Active Site of Acidic Aluminosilicate Catalysts," Nature, Vol. 309, No. 5959, pp. 589-591, 14 June 1984). The experimental conditions of the test used herein include a constant temperature of 538°C and a variable flow rate as described in detail in the Journal of Catalysis, Vol. 61, p. 395. Constraint index and the manner by which it is determined are described in U.S. Patent No. 4,016,218.

The molecular sieve catalyst employed in the process of the invention may be used in combination with a support or binder material such as, for example, a porous inorganic oxide support or a clay binder. The preferred binder is primarily silica. Other non-limiting examples of binder materials include alumina, zirconia, magnesia, thoria, titania, boria and combinations thereof, generally in the form of dried inorganic oxide gels or gelatinous precipitates. Suitable clay materials include, by way of example, bentonite and kieselguhr. The relative proportion of suitable crystalline molecular sieve to the total composition of catalyst and binder or support may be 30-90 percent by weight and is preferably 50-80 percent by weight of the composition. The composition may be in the form of an extrudate, beads, or fluidizable microspheres.

It is known to provide a molecular sieve catalyst with enhanced p-xylene selectivity and toluene conversion level by ex-situ pre-treatment with a selectivating agent (herein after referred to as "pre-selectivation") in the form of a silicon-containing compound. For pre-selectivation the silicon compound is deposited on the external surface of the catalyst by any suitable method. For example, the silicon compound may be dissolved in a solvent, mixed with the catalyst, and then dried. The silicon compound employed may be in the form of a solution, a liquid or a gas under the conditions of contact with a zeolite. Examples of methods of depositing silicon on the surface of the zeolite are found in U.S. Patents 4,090,981, 4,127,616, 4,465,886 and 4,477,583 and in EP-A-0 289 691.

Following deposition of the silicon-containing compound in pre-selectivation, the catalyst is preferably calcined. For example, the catalyst may be calcined in an oxygen-containing atmosphere, preferably air, at a rate of 0.2° to 5°C./minute to a temperature greater 300° C. but below a temperature at which the crystallinity of the zeolite is adversely affected. Generally, such temperature will be below 600°C. Preferably the temperature of calcination is within the approximate range of 350° to 550°C. The product is maintained at the calcination temperature usually for 1 to 24 hours.

According to the invention, the molecular sieve catalyst is provided with the required p-xylene selectivity and toluene conversion level by in-situ treatment with a selectivating agent (herein after referred to as "trim-selectivation"), preferably by feeding the selectivating agent simultaneously with the toluene to be disproportionated at least during a start-up phase of the reaction. The trim-selectivation phase preferably lasts for 50-300 hours, most preferably less than 170 hours. The selectivating agent is fed in an amount of 0.1-50%, preferably 0.1-20%, by weight of the toluene.

The reaction conditions during the start-up phase conveniently comprise a temperature of 100°C to 600°C, preferably 300°C to 600°C; a pressure of 100 to 14000 kPa (0 to 2000 psig), preferably 200 to 5600 kPa (15 to 800 psig); a mole ratio of hydrogen to hydrocarbons of 0 (i.e. no hydrogen is present) to 10, preferably 1 to 4 and a weight hourly space velocity (WHSV) of 0.1 to 100, preferably 0.1 to 10. Upon thermolysis, a siliceous coating is deposited on the zeolite surface, reducing or eliminating surface activity and enhancing shape-selectivity.

In an alternative embodiment, the catalyst is subjected to both pre-selectivation (as above defined) and trim-selectivation.

The compounds used as selectivating agent for the pre-selectivation and/or the trim-selectivation comprise polysiloxanes, including silicones; siloxanes; silanes, including disilanes; and alkoxysilanes having at least one silicon-hydrogen bond.

Silicone compounds which can be used in the present invention can be characterized by the general formula:

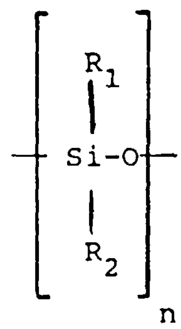

where $R_1$ is hydrogen, fluorine, hydroxy, alkyl, aralkyl, alkaryl or fluoro-alkyl. The hydrocarbon substituents generally contain from 1 to 10 carbon atoms and preferably are methyl or ethyl groups. $R_2$ is selected from the same group as $R_1$, and n is an integer of at least 2 and generally in the range of 2 to 1000. The molecular weight of the silicone compound employed is generally between 80 and 20,000 and preferably 150 to 10,000. Representative silicone compounds include dimethylsilicone, diethylsilicone, phenylmethylsilicone, methylhydrogensilicone, ethylhydrogensilicone, phenylhydrogensilicone, methylethylsilicone, phenylethylsilicone, diphenylsilicone, methyltrifluoropropylsilicone, ethyltrifluoropropysilicone, tetrachlorophenylmethyl silicone, tetrachlorophenylethyl silicone, tetrachlorophenylhydrogen silicone, tetrachlorophenylphenyl silicone, methylvinylsilicone and ethylvinylsilicone. The silicone compound need not be linear but may be cyclic as for example hexamethylcyclotrisiloxane, octamethylcyclotetrasiloxane, hexaphenylcyclotrisiloxane and octaphenylcyclotetrasiloxane, mixtures of which compounds may also be used as well as silicones with other functional groups.

Useful siloxanes or polysiloxanes include as non-limiting examples hexamethylcyclotrisiloxane, octamethylcyclotetrasiloxane, decamethylcyclopentasiloxane, hexamethyldisiloxane, octamethytrisiloxane, decamethyltetrasiloxane, hexaethylcyclotrisiloxane, octaethylcyclotetrasiloxane, hexaphenylcyclotrisiloxane and octaphenyl-cyclotetrasiloxane.

Useful silanes, disilanes or alkoxysilanes include organic substituted silanes having the general formula:

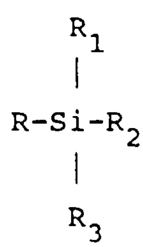

wherein R is a reactive group such as hydrogen, alkoxy, halogen, carboxy, amino, acetamide, trialkylsilyl. $R_1$, $R_2$ and $R_3$ can be the same as R or an organic radical which may include alkyl of from 1 to 40 carbon atoms, alkyl or aryl carboxylic acid wherein the organic portion of the alkyl contains 1 to 30 carbon atoms and the aryl group contains 6 to 24 carbon atoms, aryl groups of 6 to 24 carbons which may be further substituted, alkylaryl and arylalkyl groups containing 7 to 30 carbon atoms. Preferably, the alkyl group of an alkyl silane is between 1 and 4 carbon atoms in chain length. Mixtures may also be used.

The silanes or disilanes include, as non-limiting examples, dimethylphenylsilane, phenytrimethylsilane, triethylsilane and hexamethyldisilane. Useful alkoxysilanes are those with at least one silicon-hydrogen bond.

While not wishing to be bound by theory, it is believed that the advantages of the present invention are obtained by rendering acid sites on the external surfaces of the catalyst substantially inaccessible to reactants while increasing catalyst tortuosity. Acid sites existing on the external surface of the catalyst are believed to isomerize the para-xylene exiting the catalyst pores back to an equilibrium level with the other two isomers thereby reducing the amount of para-xylene in the xylenes to only about 24%. By reducing the availability of these acid sites to the para-xylene exiting the pores of the catalyst, the relatively high level of para-xylene can be maintained. It is believed that the selectivating agents of the present invention block or otherwise render these external acid sites unavailable to the para-xylene by chemically modifying said sites.

Preferably, the kinetic diameter of the selectivating agent is larger than the zeolite pore diameter, in order to avoid reducing the internal activity of the catalyst.

It is believed that the presence of hydrogen in the reaction zone is important in order to maintain the desired high yields of para-xylene when a silicone compound is used as the trim-selectivating agent.

The processes disclosed herein greatly enhance the efficiency of para-xylene production. For example, a para-xylene purity of greater than 90%, preferably at least 95%, based on all $C_8$ products can be attained with a toluene conversion of greater than 15%, preferably at least 20%, and most preferably at least 25%. Moreover, the ortho-xylene isomer can be reduced to not more than about 0.5% of the total xylene content while the meta-xylene isomer can be reduced to less than about 5% of the total xylene content. In addition, when the reaction system is properly treated, such as by deposition of platinum on the molecular sieve, the presence of ethylbenzene can be significantly reduced, e.g., to less than about 2% of the $C_8$ product.

The feedstock used in the process of the invention preferably comprises 50% to 100%, more preferably at least 80%, by weight toluene. Other compounds such as benzene, xylenes, and trimethylbenzenes may also be present in the feedstock without adversely affecting the process.

The toluene feedstock may also be dried, if desired, in a manner which will minimize moisture entering the reaction zone. Methods known in the art suitable for drying the toluene charge for the present process are numerous. These methods include percolation through any suitable dessicant, for example, silica gel, activated alumina, molecular sieves or other suitable substances, or the use of liquid charge dryers.

Operating conditions for the toluene disproportionation process of the present invention include a temperature of 350 - 540°C, preferably greater than 400°C, a pressure of 100 to 35000 kPa (atmospheric to 5000 psig), preferably 800 to 7000 kPa (100 to 1000 psig), a WHSV of 0.1-20, preferably 2-4, and a hydrogen to hydrocarbon mole ratio of 0.1-20, preferably 2-4. This process may be conducted in either fixed- or fluid-bed mode with attendant benefits of either operation readily obtainable.

The effluent is separated and distilled to remove the desired product, i.e., para-xylene, plus other by-products. The unreacted reactant, i.e. toluene, is preferably recycled for further reaction. The benzene is a valuable co-product.

In accordance with a preferred embodiment of the present invention, the catalyst is further modified in order to reduce the amount of undesirable by-products, particularly ethylbenzene. The state of the art is such that the reactor effluent from toluene disproportionation typically contains about 0.5% ethylbenzene by-product. Upon distillation of the reaction products, the level of ethylbenzene in the $C_8$ fraction often increases to 3-4 percent. This level of ethylbenzene is unacceptable for polymer grade p-xylene since ethylbenzene in the $C_8$ product, if not removed, degrades the quality of fibers ultimately produced from the p-xylene product. Consequently, ethylbenzene content must be kept low. The specification for ethylbenzene in the $C_8$ product has been determined by industry to be less than 0.3%. The ethylbenzene can be substantially removed by isomerization or by superfractionation processes. Removal of the ethylbenzene by conventional isomerization would be impractical with the present invention since the xylene stream, which comprises greater than 90% para-xylene, would be concurrently isomerized to equilibrium xylenes reducing the amount of para-xylene in this xylene stream to about 24%. Moreover, it is known that the alternative procedure of removing the ethylbenzene by superfractionation is extremely expensive.

In order to avoid the need for downstream ethylbenzene removal, the level of ethylbenzene by-product is advantageously reduced by incorporating a hydrogenation-dehydrogenation function in the catalyst via addition of a metal compound such as platinum. While platinum is the preferred metal, other metals such as palladium, nickel, copper, cobalt, molybdenum, rhodium, ruthenium, silver, gold, mercury, osmium, iron, zinc, cadmium, and mixtures thereof may be utilized. The metal may be added by cation exchange, in amounts of 0.01 - 2%, typically about 0.5%. The metal must be able to enter the pores of the catalyst in order to survive a subsequent calcination step. For example, a platinum modified catalyst can be prepared by first adding to the catalyst a solution of ammonium nitrate in order to convert the catalyst to the ammonium form, and subsequently, an aqueous solution of tetraamine platinum(II) nitrate to increase activity. The catalyst can then be filtered, washed with water and calcined at temperatures of 250° to 500°C.

The invention will now be more particularly described with reference to the Examples and the accompanying drawings, in which:

Figure 1 is a graph comparing xylene para-selectivity and toluene conversion over a silicone trim-selectivated ZSM-5 catalyst in the presence of hydrogen (Example 1) or nitrogen as a function of stream time,

Figure 2 is a graph similar to Figure 1 and provides results of a hydrogen co-feed at the slightly lower temperature used in Example 2,

Figure 3 is also a graph similar to Figure 1 and shows results obtained in the absence of a hydrogen co-feed (Example 3),

Figure 4 is a graph showing para-xylene and toluene conversion as a function of time on stream.

Figure 5 depicts para-selectivity and conversion rates for a zeolite which has been pre-selectivated with 10% $SiO_2$, and

Figure 6 provides para-selectivity and conversion rates for a zeolite which has been pre-selectivated with 5% $SiO_2$.

## EXAMPLE 1

Toluene disproportionation was carried out in a fixed-bed reactor using 2 grams of a silica bound HZSM-5 catalyst having a silica/alumina ratio of 26, a crystal size of 0.1 micron, an Alpha Value of 731. The feed to the reactor was toluene containing 1% silicone compound having a phenylmethyl silicone to dimethyl silicone ratio of 1:1. Operating conditions were 4.0 WHSV, 480°C, 3550 kPa (500 psig), and a hydrogen/ hydrocarbon ratio of 2. Table 1 summarizes toluene conversion and para-xylene selectivity as a function of time on stream during and after trim-selectivation.

TABLE 1

| Time on Stream, hr | Conversion, wt% | para-Xylene in Xylenes, wt% |
|---|---|---|
| 1 | 56 | 22 |
| 6 | 57 | 21 |
| 22 | 51 | 24 |
| 46 | 42 | 39 |
| 98 | 36 | 70 |
| 143 | 28 | 86 |
| 170 | 25 | 89 |
| 174* | 25 | 91 |
| 342* | 25 | 91 |

* Silicone co-feed discontinued.

It is noteworthy that the silicone trim-selectivation substantially increased para-xylene selectivity from an initial 22% to over 90%. At 174 hours on stream the feed was changed to 100% toluene, i.e., the silicone co-feed was discontinued. Over the following one week test period, toluene conversion remained constant at 25% and para-xylene selectivity remained constant at 91%.

The above results are illustrated by Figure 1, which also includes the results of conducting the selectivation in the presence of nitrogen, rather than hydrogen. In the presence of nitrogen, the catalyst deactivated rapidly and conversion quickly approached zero.

## EXAMPLE 2

The toluene disproportionation process of Example 1 was repeated at 4.0 WHSV, 446°C, 3550 kPa (500 psig), and a hydrogen/hydrocarbon ratio = 2. Table 2 summarizes toluene conversion and para-xylene selectivity as a function of time on stream.

TABLE 2

| Time on Stream, hr | Conversion, wt% | p-Xylene in Xylenes, wt% |
|---|---|---|
| 1 | 44 | 29 |
| 25 | 42 | 34 |
| 47 | 37 | 58 |
| 94 | 31 | 86 |
| 143 | 29 | 93 |
| 176 | 27 | 96 |

TABLE 2 (continued)

| Time on Stream, hr | Conversion, wt% | p-Xylene in Xylenes, wt% |
|---|---|---|
| 199 | 26 | 97 |
| 223 | 25 | 97 |
| 239* | 25 | 97 |

* Silicone co-feed discontinued.

Silicone trim-selectivation increased para-xylene selectivity from 24% (thermodynamic value) to a high 97% at 25% toluene conversion. When the silicone co-feed was discontinued, the para-xylene selectivity and toluene conversion were unchanged at 97% and 25%, respectively. The results are illustrated by Figure 2.

**EXAMPLE 3**

The toluene disproportionation process of Example 1 was repeated at 4.0 WHSV, 420°C, 100 kPa (0 psig), and hydrogen/hydrocarbon ratio = 0. Table 3 and Figure 3 summarize toluene conversion and para-xylene selectivity as a function of time on stream. Note that the conversion drops to essentially zero at 184 hours on stream in contrast to operation in the presence of hydrogen where at 184 hours on stream conversion has stabilized at 25%.

TABLE 3

| Time on Stream,. hr | Conversion,. wt% | para-Xylene in Xylenes, wt% |
|---|---|---|
| 1 | 14 | 27 |
| 48 | 8 | 51 |
| 96 | 2 | 82 |
| 136 | 1 | 93 |
| 184 | 0.1 | 97 |

**EXAMPLE 4**

Toluene disproportionation over $SiO_2$-HZSM-5 was carried out using 1% octamethylcyclotetrasiloxane in a toluene feed as the trim-selectivating agent. Operating conditions were 446°C, 3550 kPa (500 psig), 4.0 WHSV, and $H_2$/HC=2. Table 4 summarizes the results.

TABLE 4

| Time on Stream, Hrs. | p-Xylene/Xylenes, wt% | Toluene Conversion, wt% |
|---|---|---|
| 0 | 25 | 40 |
| 24 | 88 | 23 |
| 46 | 95 | 18 |
| 71 | 98 | 15 |

**EXAMPLE 5**

Toluene disproportionation with trim-selectivation as in Example 4 was carried out using hexamethyldisiloxane (HMDS). Table 5 and Figure 4 summarize the results.

TABLE 5

| Time on Streams, hrs. | p-Xylene in Xylenes, wt% | Toluene Conversion, wt% |
|---|---|---|
| 1 | 28 | 47 |
| 2 | 56 | 42 |
| 4 | 80 | 37 |
| 14 | 95 | 33 |
| 24 | 98 | 28 |
| 47 | 99 | 20 |

TABLE 5 (continued)

| Time on Streams, hrs. | p-Xylene in Xylenes, wt% | Toluene Conversion, wt% |
|---|---|---|
| 54 | 99 | 18 |

Figure 4 illustrates the high p-xylene selectivity and toluene conversion over 350 hours on stream. The toluene conversion remained at about 18 - 20% with a p-xylene selectivity of 99% for an extended period of time. HMDS was discontinued after about 50 hours.

**EXAMPLES 6-14**

The process of Example 4 was repeated out with trim-selectivation using the siloxanes listed in Table 6. Operating conditions were 446°C, 3550 kPa (500 psig), 4.0 WHSV and $H_2$/HC = 2. The results after 24 hours are shown in Table 6.

TABLE 6

| Ex. | Siloxanes | p-Xylene/Xylenes, wt.% | Toluene Conversion wt.% |
|---|---|---|---|
| 6 | Methylhydrocyclosiloxanes | 89 | 13 |
| 7 | Hexamethylcyclotrisiloxane | 84a | 20 |
| 8 | 1,3,5-Trimethyl-1,3,5-triphenylcyclotrisiloxane | 85 | 31 |
| 9 | Octamethylcyclotetrasiloxane | 88 | 23 |
| 10 | Decamethylcyclopentasiloxane | 90 | 28 |
| 11 | Decamethyltetrasiloxane | 98 | 24 |
| 12 | Hexamethyldisiloxane | 98 | 24 |
| 13 | 1,1,3,3,5,5-Hexamethyltrisiloxane | 96 | 14 |
| 14 | Octamethyltrisiloxane (after 41 hrs) | 81[a] | 20 |

[a] - continuation of the selectivation beyond the cited time periods brings the p-xylene selectivity to above 90% with toluene conversion of at least 15%.

**EXAMPLES 15-19**

Trim-selectivations as in Examples 4 and 5 were carried out with the silanes listed in Table 7. Operating conditions were 446°C, 3550 kPa (500 psig), 4.0 WHSV and $H_2$/HC = 2. The results after 24 hours are shown in Table 7.

TABLE 7

| Ex. | Siloxanes | p-Xylene/ Xylenes, wt.% | Toluene Conversion wt.% |
|---|---|---|---|
| 15 | Diphenylsilane | 96 | 15 |
| 16 | Dimethylphenylsilane | 97 | 19 |
| 17 | Phenyltrimethylsilane | 86[a] | 19 |
| 18 | Triethylsilane | 85[a] | 21 |
| 19 | Hexamethyldisilane | 95 | 23 |

[a] - continuation of the selectivation beyond 24 hours brings the p-xylene selectivity to above 90%.

**COMPARATIVE EXAMPLES 20-22**

For comparison purposes, the compounds listed in Table 8 were tested as in Examples 6-19 with results shown in Table 8.

TABLE 8

| Ex. | Siloxanes | p-Xylene/Xylenes, wt.% | Toluene Conversion wt.% |
|---|---|---|---|
| 20 | Tetra-(n-butyl) -orthosilicate | 36 | 2 |
| 21 | Tetra-ethyl-orthosilicate | 38 | 3 |

TABLE 8 (continued)

| Ex. | Siloxanes | p-Xylene/Xylenes, wt.% | Toluene Conversion wt.% |
|---|---|---|---|
| 22 | Tetra-(2-ethylhexyl)-orthosilicate | 33 | 1 |

**EXAMPLE 23**

A silica pre-selectivated ZSM-5 catalyst was prepared by adding 5.00 g HZSM-5 to 1.26 g phenylmethylpolysiloxane dissolved in 40 cc hexane. The solvent was distilled and the catalyst was air calcined at 1°C/min to 538°C, then 6 hours at 538°C. The pre-selectivated catalyst contained a nominal 10% added silica.

Silicone trim selectivation of the 10% $SiO_2$-HZSM-5 was carried out at 446°C, 3550 kPa (500 psig), 4.0 WHSV, and hydrogen/hydrocarbon ratio = 2. Table 9 and Figure 5 show toluene conversion and para-xylene selectivity for 10% $SiO_2$-HZSM-5 as a function of time on stream.

TABLE 9

| Silicone Selectivation of 10% $SiO_2$-HZSM-5 | | |
|---|---|---|
| Time on Stream, hrs | Toluene Conversion, wt% | Para-xylene in Xylenes, wt% |
| 2 | 25 | 33 |
| 4 | 24 | 43 |
| 6 | 23 | 72 |
| 8 | 21 | 84 |
| 10 | 21 | 89 |
| 15 | 19 | 94 |
| 20 | 18 | 96 |
| 28 | 18 | 98 |

The silicone trim selectivation substantially increased para-xylene selectivity from 33% to 98% over 28 hours on stream. Feed was then changed to 100% toluene. Over the next ten hours the selectivity increased to 99% at 16% conversion. To further increase conversion, the temperature was increased to 457°C and shortly thereafter to 468°C. The conversion rose to 21%, then decreased slightly to 20% over the next 80 hours. The para-xylene selectivity increased from 99.2% to 99.6% over the same 80 hours.

Compared to the HZSM-5 of Example 1, the 10% $SiO_2$-HZSM-5 (pre-selectivated) catalyst of Example 23 showed a substantially higher selectivation rate. For pre-selectivated HZSM-5, 89% para-xylene selectivity was achieved after only 10 hours on stream (17 times faster than the 170 hours for the HZSM-5 parent). Also, the time needed to reach optimum para-selectivation, 1 day for pre-selectivated HZSM-5 compared to 1 week for HZSM-5, was shorter despite the higher selectivation temperature for HZSM-5(480°C vs. 446°C).

The total phenylmethyl silicone consumption was 6.80 g silicone per g HZSM-5 and 1.42 gram of silicone per gram of pre-selectivated HZSM-5. Thus trim-selectivation of the pre-selectivated HZSM-5 consumed nearly five (4.79) times less silicone than in the case of the non-pre-selectivated a catalyst.

**EXAMPLE 24**

Example 23 was repeated but with the pre-selectivated catalyst containing only 5% added silica. Table 10 and Figure 6 show toluene conversion and para-xylene selectivity for 5% $SiO_2$-HZSM-5 as a function of time on stream.

TABLE 10

| Silicone Selectivation of 5% $SiO_2$-HZSM-5 | | |
|---|---|---|
| Time on Stream, hrs | Toluene Conversion, wt% | para-xylene in Xylenes, wt% |
| 2 | 41 | 25 |
| 4 | 41 | 27 |
| 5 | 38 | 36 |
| 7 | 35 | 54 |
| 14 | 31 | 83 |
| 21 | 27 | 95 |

TABLE 10 (continued)

| Silicone Selectivation of 5% $SiO_2$-HZSM-5 | | |
|---|---|---|
| Time on Stream, hrs | Toluene Conversion, wt% | para-xylene in Xylenes, wt% |
| 26 | 25 | 98 |

Silicone trim-selectivation substantially increased para-xylene selectivity from 25% to 98% over 26 hours on stream. Compared to 10% $SiO_2$-HZSM-5, the 5% $SiO_2$ catalyst showed consistently higher conversion over the one day selectivation time. Feed was then changed to 100% toluene. Over the next 6 hours the selectivity increased to 99% at 24% conversion, temperature was increased to 468°C and WHSV was decreased to 3. Conversion increased to 27%, then gradually decreased to and remained constant at 21% for 6 days (146 hours). Correspondingly, the para-xylene selectivity was initially unchanged at 99% then gradually increased to and remained constant at 99.6%-99.9% for 6 days when the run was arbitrarily terminated.

**EXAMPLE 25**

A 0.05% Pt-10% $SiO_2$-HZSM-5 catalyst was prepared by adding 2.50 g of the 10% $SiO_2$-HZSM-5 prepared in Example 25 to 12.5 cc 1M ammonium nitrate solution. After 1.5 hours, a solution of 0.0025 g tetraamine platinum (II)nitrate in approximately 0.5 cc water was added. After standing overnight the catalyst was filtered, washed with water, and air calcined at 5°C/min to 350°C, then 3 hours at 350°C.

Toluene disproportionation was carried out over 2.00 g of the resultant catalyst at 446°C, 3550 kPa (500 psig), 4 WHSV, and a hydrogen/hydrocarbon mole ration of 2.0. Table 11 shows the product distribution compared to that of Pt-free silica-modified HZSM-5 from Example 23 tested under the same operating conditions. At similar toluene conversion, the ethylbenzene product was reduced by nearly a factor of 12 using the Pt-catalyst. The undesirable $C_9^+$ aromatics product also was reduced by nearly a factor of 2.

TABLE 11

| Component, wt% | Pt-$SiO_2$-HZSM-5 | $SiO_2$-HZSM-5 |
|---|---|---|
| Benzene | 45.84 | 41.65 |
| Ethylbenzene | 0.05 | 0.59 |
| Xylenes | 43.12 | 55.98 |
| $C_{9+}$ Aromatics | 0.99 | 1.78 |
| | 100.00 | 100.00 |
| Ethylbenzene in $C_8$, wt. | 0.10 | 1.18 |
| p-Xylene in Xylenes, wt% | 25.8 | 29.8 |
| Toluene Conversion, wt% | 35 | 34 |

**EXAMPLE 26**

The catalyst of Example 25 was treated in situ with a 1% solution of phenymethylpolysiloxane in toluene at 446°C, 3550 kPa (500 psig), 4 WHSV, and a hydrogen/hydrocarbon mole ratio of 2.0. After 32 hours on stream the feed was changed to 100% toluene. Table 12 shows the product distribution compared to that of Pt-free, siloxane treated, silica-modified HZSM-5 tested under the same operating conditions.

TABLE 12

| Component, wt% | Pt-$SiO_2$-HZSM-5 | $SiO_2$-HZSM-5 |
|---|---|---|
| Benzene | 46.62 | 38.43 |
| Ethylbenzene | 0.33 | 1.18 |
| Xylenes | 52.35 | 58.56 |
| $C_9$+ Aromatics | 0.70 | 1.83 |
| | 100.00 | 100.00 |
| Ethylbenzene in $C_8$, wt% | 0.63 | 1.98 |
| p-Xylene in Xylenes, wt% | 98.4 | 98.7 |
| Toluene Conversion, wt% | 25 | 22 |

At similar toluene conversion, the ethylbenzene product was reduced by a factor of 3.6 using the Pt-catalyst while the p-xylene selectivities remained very high at 98.4%-98.7%. The undesirable $C_9$+ aromatics product was also reduced by nearly a factor of 3.

The results of Examples 27-29 which are reported in Table 13, indicate the beneficial effect on ethylbenzene in the product stream by the addition of platinum to the catalytic molecular sieve.

## EXAMPLE 27

Silicone trim-selectivation of a 10% $SiO_2$-HZSM-5 was carried out using 1% phenylmethyl silicone in a toluene feed at 446°C, 3550 kPa (500 psig), 4.0 WHSV, and a hydrogen/hydrocarbon ratio = 2. At 31 hours on stream the feed was changed to 100% toluene. At 52 hours on stream the temperature was increased to 468°C and at 165 hours the WHSV was lowered to 3.0. The data at 39 days on stream are shown in column 1 of Table 13.

## EXAMPLE 28

Silicone selectivation of a 0.025%Pt 10%$SiO_2$-HZSM-5 was carried out using 1% phenylmethyl silicone in a toluene feed at 446°C, 3550 kPa (500 psig), 4.0 WHSV, and a hydrogen/hydrocarbon ratio = 2. At 56 hours on stream the feed was changed to 100% toluene. At 73 hours on stream the temperature was increased to 468°C. The data at 7 days on stream are shown in column 2 of Table 13.

## EXAMPLE 29

Silicone selectivation of a nitric acid activated 0.05% Pt 10% $SiO_2$-HZSM-5 was carried out using 1% phenylmethyl silicone in a toluene feed at 446°C, 3550 kPa (500 psig), 4.0 WHSV, and a hydrogen/hydrocarbon ratio = 2. At 27 hours on stream the feed was changed to 100% toluene. Temperature, WHSV, and hydrogen/hydrocarbon ratio were varied during the run. The data at 13 days on stream are shown in column 3 of Table 13.

TABLE 13

| | Silicone | Silicone/Pt | |
|---|---|---|---|
| | Ex. 27 | Ex. 28 | Ex. 29 |
| Reaction Conditions | | | |
| Temperature, °C | 468 | 468 | 431 |
| Pressure, psig | 500 | 500 | 500 |
| $H_2$/HC | 2 | 2 | 8 |
| WHSV | 3 | 4 | 4 |
| Time on Stream, days | 39 | 7 | 13 |
| Toluene Conversion, wt% | 23 | 20 | 21 |
| Products, wt% | | | |
| $C_5^-$ | 2.5 | 2.5 | 2.5 |
| Benzene | 43.0 | 43.6 | 47.2 |
| Ethylbenzene | 1.9 | 0.2 | 0.1 |
| Xylenes | 50.4 | 53.1 | 50.0 |
| Ethyltoluenes | 1.9 | 0.5 | 0.2 |
| $C_{10}^+$ | 0.3 | 0.1 | 0.0 |
| | 100.0 | 100.0 | 100.0 |
| p-Xylene | 99.7 | 98.7 | 99.7 |
| m-Xylene | 0.3 | 1.3 | 0.3 |
| o-Xylene | tr. | tr. | tr. |
| | 100.0 | 100.0 | 100.0 |
| Benzene/Xylenes, m/m | 1.2 | 1.1 | 1.3 |
| p-XylenePurity, wt% | 97.8 | 98.3 | 99.5 |

Example 27-29 indicate that the levels of ethylbenzene in the reaction products of the present invention can be reduced by using a catalytic molecular sieve with a hydrogenation/dehydrogenation function such as platinum incorporated into the catalytic molecular sieve. The level of ethylbenzene in the product stream is preferably at a commercially acceptable level of not greater than 0.3%, and is most preferably not greater than about 0.2%.

As stated above, the present invention advantageously provides a product stream having a high para-xylene purity with respect to the other $C_8$ products. Table 14 provides the relative proportions of para-xylene to various combinations of other products.

TABLE 14

| Comparison of Product Parameters | | | | |
|---|---|---|---|---|
| | CATALYST | | | |
| Parameter | Silicone | Silicone/Pt | | Calculated Equilibrium Value |
| | Ex. 27 | Ex. 28 | Ex. 29 | |
| p-Xylene/EB | 26.4 | 262 | 498 | 2.5 |
| p-Xyl/EB+m,o-xyl (other $C_8$) | 23.9 | 58.2 | 166 | 2.5 |
| p-Xyl/EB+m,o-Xyl+$C_9$ (other $C_8$+$C_9$) | 12.6 | 37.4 | 99.6 | 1.6 |
| p-Xylene purity (in all $C_8$s), wt% | 95.7 | 98.3 | 99.5 | 71.8 |
| p-Xylene yield (based on all products and toluene), wt% | 10.6 | 10.6 | 10.2 | 11.9 |

## Claims

1. A toluene disproportionation process comprising contacting a feed stream comprising toluene with a molecular sieve catalyst to produce a product comprising xylene and benzene, characterized by contacting the catalyst during the process with an alkoxysilane having at least one silicon-hydrogen bond, a polysiloxane, a siloxane, a silane and/or a disilane added to said stream so as to yield a single-pass product whose C8 fraction comprises at least 90% paraxylene at a toluene conversion of at least 15%.

2. The process according to claim 1, wherein said feed stream comprises at least 80% by weight of toluene and at least 0.1% by weight of the organosilicon compound.

3. The process according to claim 1 or claims 2 wherein the molecular sieve is also treated with an organosilicon compound prior to contacting said feed stream.

4. The process according to any preceding claim wherein said molecular sieve catalyst has a constraint index of 1-20.

5. The process according to any preceding claim wherein said contacting is effected at a temperature of 350° to 540°C, a pressure of 100 to 35000 kPa (atmospheric to 5000 psig), a WHSV of 0.1 to 20, and a hydrogen to hydrocarbon molar ratio of 0.1 to 20.

6. The process according to any preceding claim wherein said molecular sieve catalyst includes a metal hydrogenation-dehydrogenation component.

7. The process according to claim 6 wherein said metal comprises platinum.

## Patentansprüche

1. Verfahren zur Disproportionierung von Toluol, das den Kontakt eines Beschickungsstroms, der Toluol umfaßt, mit einem Molekularsieb-Katalysator umfaßt, wodurch ein Produkt hergestellt wird, das Xylol und Benzol umfaßt, gekennzeichnet durch den Kontakt des Katalysators während des Verfahrens mit einem Alkoxysilan mit mindestens einer Silicium-Wasserstoff-Bindung, einem Polysiloxan, einem Siloxan, einem Silan und/oder einem Disilan, das dem Strom zugesetzt wird, wodurch in einem Durchlauf ein Produkt erhalten wird, dessen $C_8$-Fraktion mindestens 90% p-Xylol umfaßt, bei einer Toluolumwandlung von mindestens 15%.

2. Verfahren nach Anspruch 1, wobei der Beschickungsstrom mindestens 80 Gew.-% Toluol und mindestens 0,1

Gew.-% der Organosiliciumverbindung umfaßt.

3. Verfahren nach Anspruch 1 oder Anspruch 2, wobei das Molekularsieb vor dem Kontakt mit dem Beschickungsstrom auch mit einer Organosiliciumverbindung behandelt wird.

4. Verfahren nach einem der vorstehenden Ansprüche, wobei der Molekularsieb-Katalysator einen Zwangsindex von 1 bis 20 hat.

5. Verfahren nach einem der vorstehenden Ansprüche, wobei der Kontakt bei einer Temperatur von 350 bis 140°C, einem Druck von 100 bis 35000 kPa (Atmosphärendruck bis 5000 psig), einer WHSV von 0,1 bis 20 und einem Molverhältnis von Wasserstoff/Kohlenwasserstoff von 0,1 bis 20 erfolgt.

6. Verfahren nach einem der vorstehenden Ansprüche, wobei der Molekularsieb-Katalysator eine Hydrierungs/Dehydrierungs-Komponente aus einem Metall umfaßt.

7. Verfahren nach Anspruch 6, wobei das Metall Platin umfaßt.

**Revendications**

1. Un procédé de disproportionation du toluène comprenant la mise en contact d'un courant d'alimentation comprenant du toluène avec un catalyseur de type tamis moléculaire pour fournir un produit comprenant du xylène et du benzène, caractérisé par la mise en contact du catalyseur pendant le procédé avec un alcoxysilane ayant au moins une liaison silicium-hydrogène, un polysiloxane, un siloxane, un silane et/ou un disilane ajouté audit courant de façon à fournir un produit après un passage unique dont la fraction en $C_8$ comprend au moins 90% de para-xylène avec une conversion du toluène d'au moins 15%.

2. Un procédé suivant la revendication 1, dans lequel ledit courant d'alimentation comprend au moins 80% en poids de toluène et au moins 0,1% en poids du composé organosilicique.

3. Un procédé suivant les revendications 1 ou 2, dans lequel le tamis moléculaire est aussi traité par un composé organosilicique avant la mise en contact avec ledit courant d'alimentation.

4. Un procédé suivant l'une quelconque des revendications précédentes, dans lequel ledit catalyseur de type tamis moléculaire a un indice de contrainte de 1 à 20.

5. Un procédé suivant l'une quelconque des revendications précédentes, dans lequel ladite mise en contact est effectuée à une température de 350°C à 540°C, une pression de 100 à 35000 kPa (pression atmosphérique à 5000 psig), une WHSV de 0,1 à 20, et un rapport molaire d'hydrogène à hydrocarbure de 0,1 à 20.

6. Un procédé suivant l'une quelconque des revendications précédentes, dans lequel ledit catalyseur de type tamis moléculaire comprend un composant métallique d'hydrogénation/déshydrogénation.

7. Procédé suivant la revendication 6, dans lequel ledit métal comprend le platine.

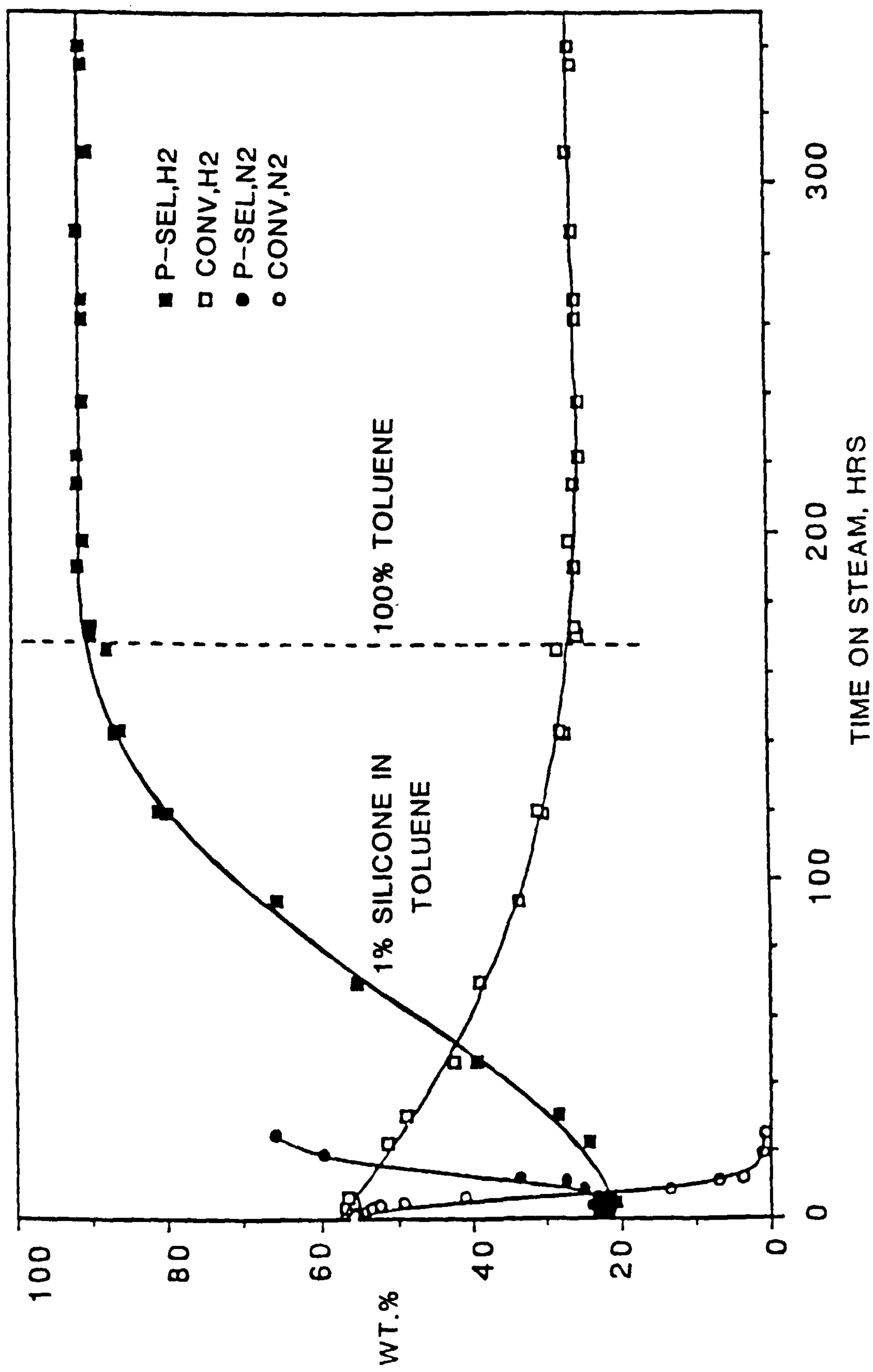
P-SEL,H2
CONV,H2
P-SEL,N2
CONV,N2
1% SILICONE IN TOLUENE
100% TOLUENE
WT.%
100
80
60
40
20
0
0
100
200
300
TIME ON STEAM, HRS

FIG. I

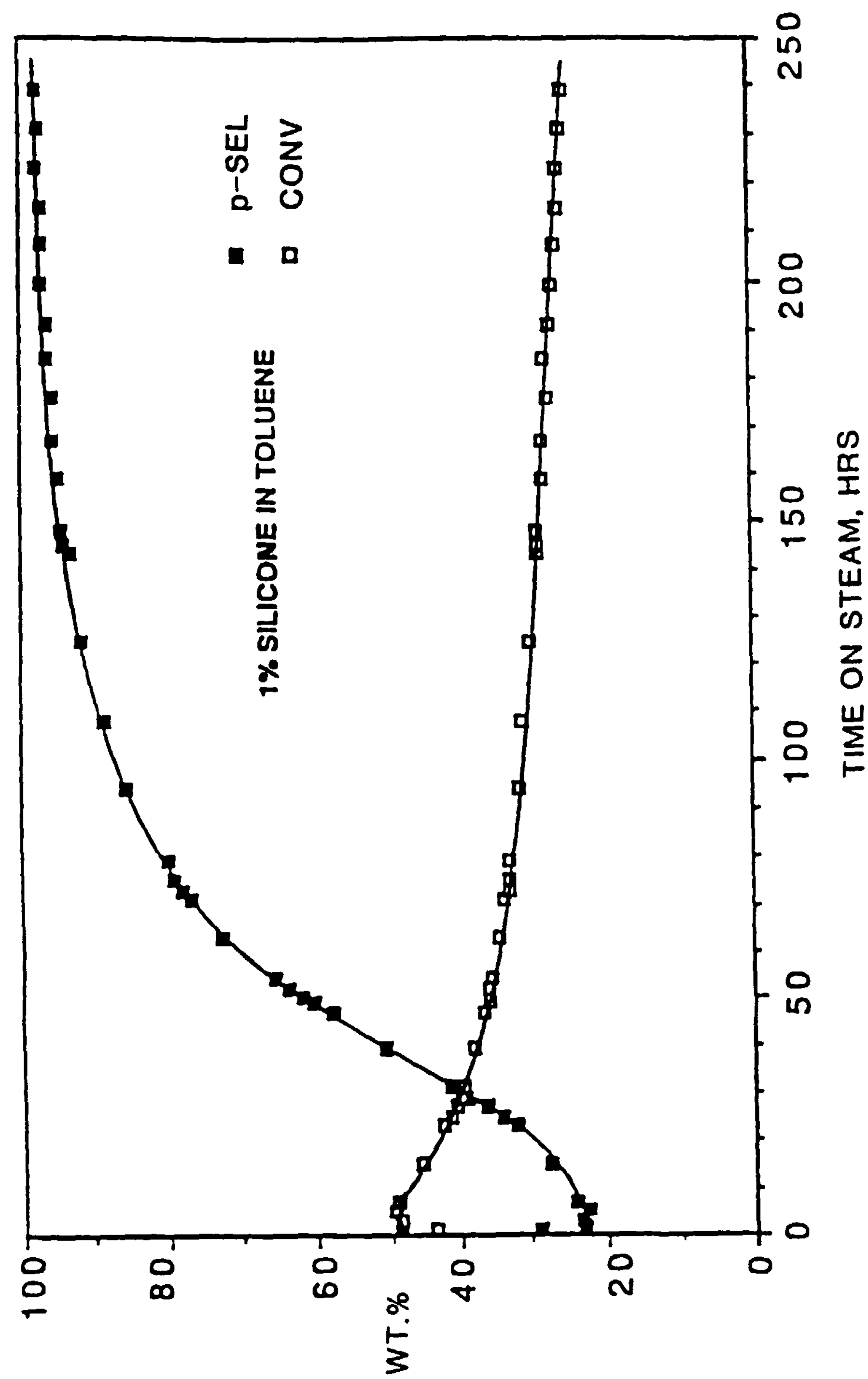

1% SILICONE IN TOLUENE
p-SEL
CONV
WT.%
100
80
60
40
20
0
50
100
150
200
250
TIME ON STEAM, HRS


FIG. 2

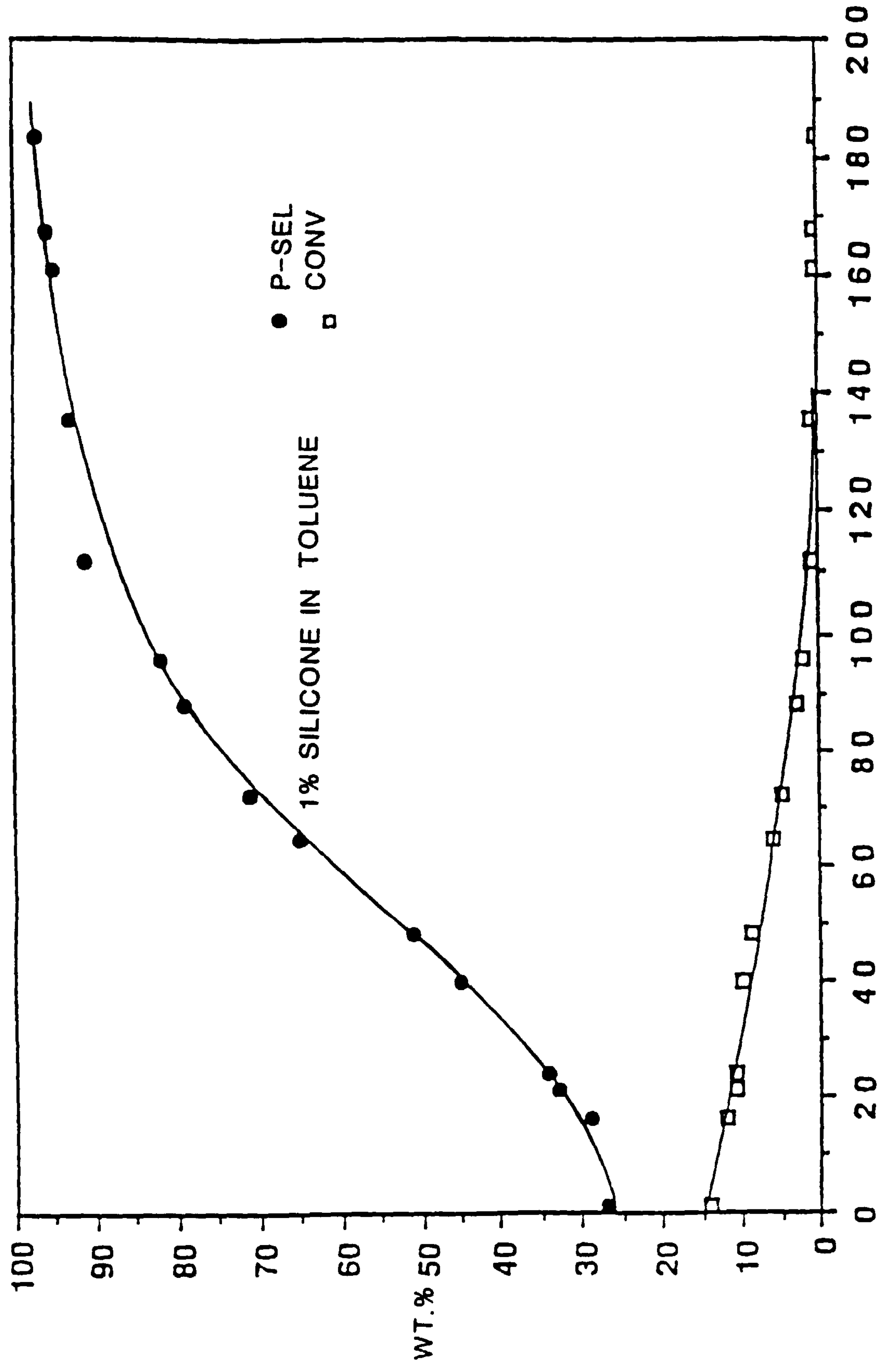
1% SILICONE IN TOLUENE
P-SEL
CONV
WT.%
TIME ON STEAM, HRS
0
20
40
60
80
100
120
140
160
180
200
0
10
20
30
40
50
60
70
80
90
100

FIG. 3

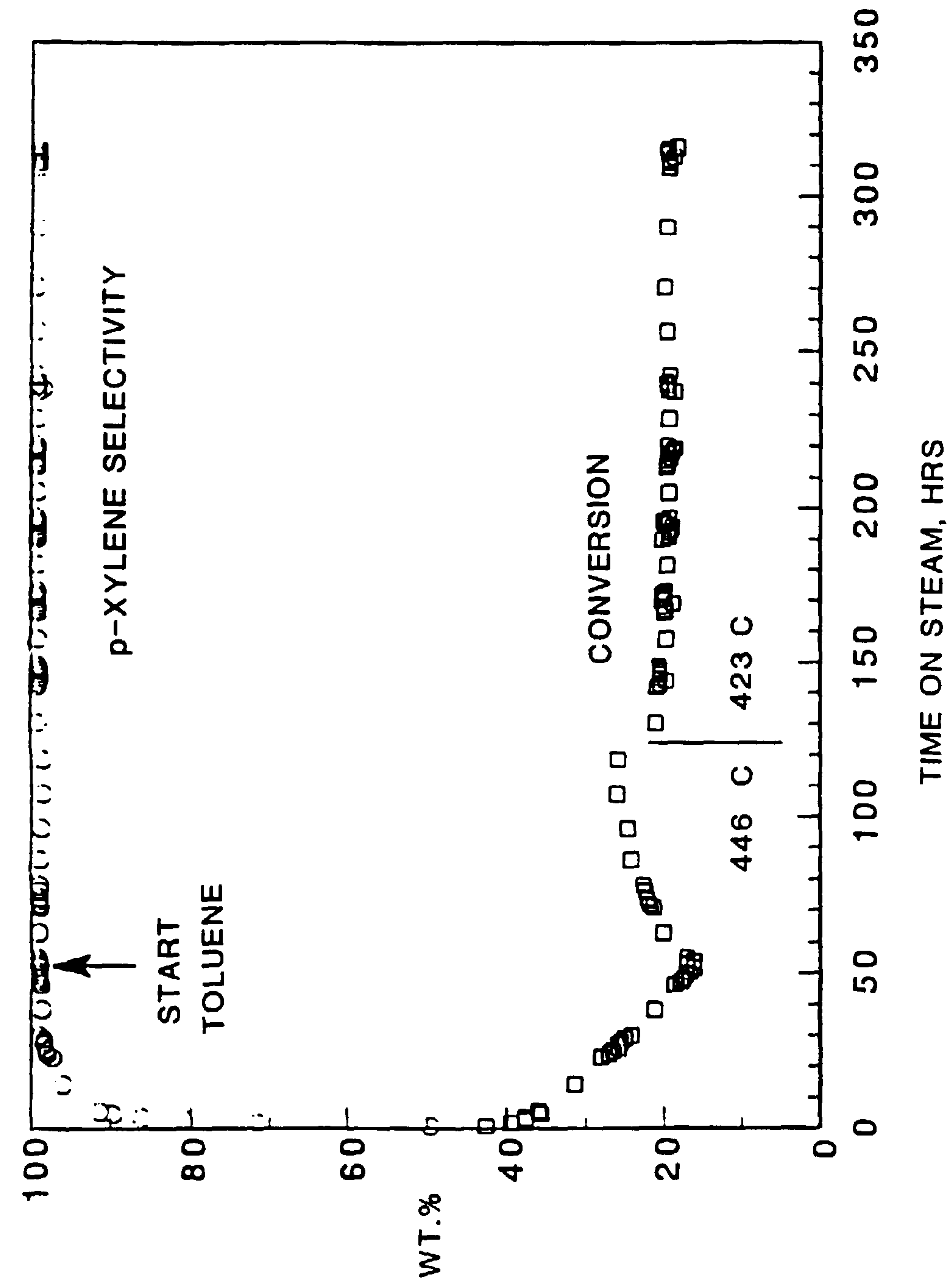
p-XYLENE SELECTIVITY
START TOLUENE
CONVERSION
446 C
423 C
WT.%
TIME ON STEAM, HRS
0
20
40
60
80
100
0
50
100
150
200
250
300
350

FIG. 4

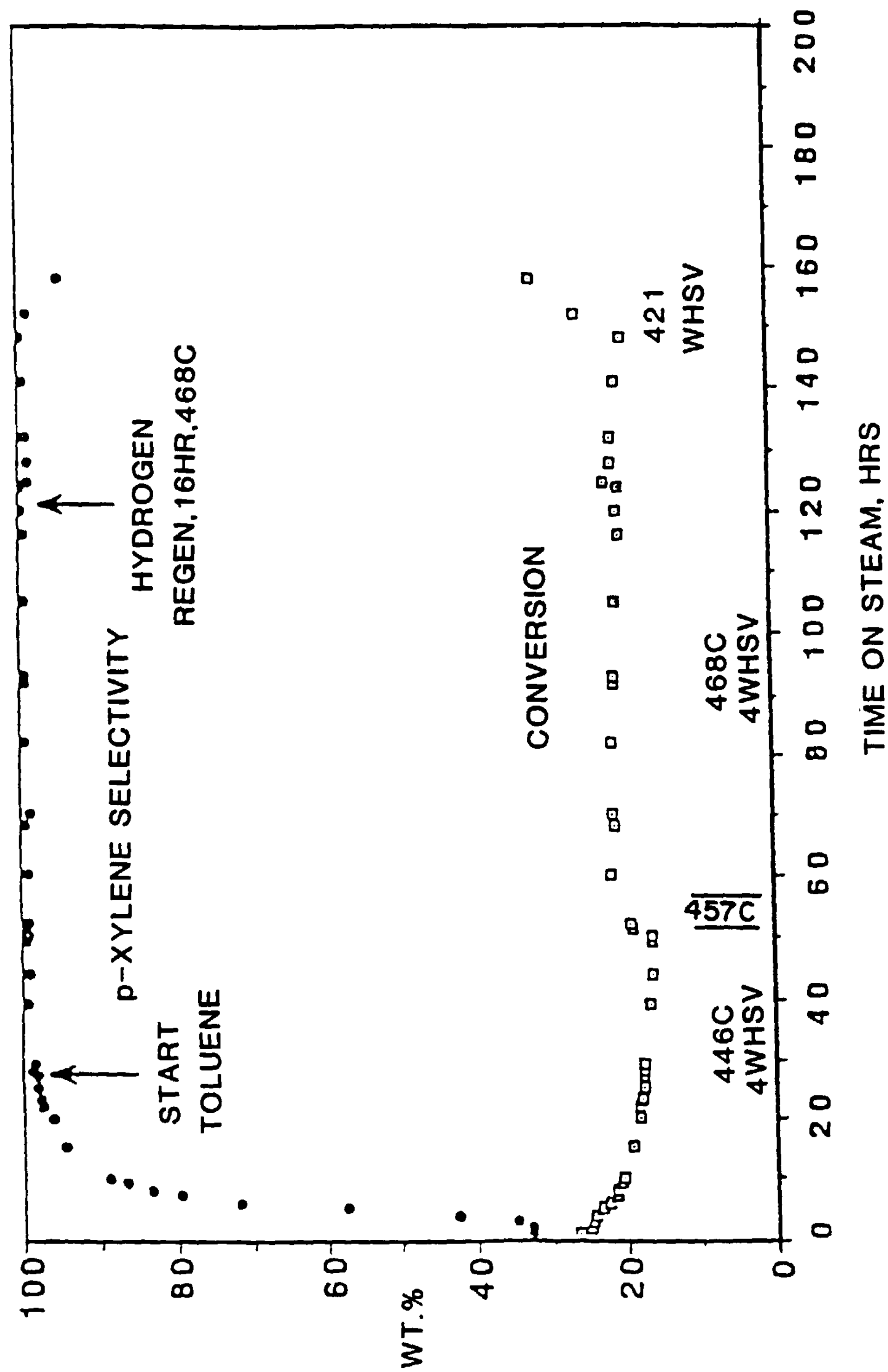
WT.%
0
20
40
60
80
100
TIME ON STEAM, HRS
0
20
40
60
80
100
120
140
160
180
200
p-XYLENE SELECTIVITY
START
TOLUENE
HYDROGEN
REGEN,16HR,468C
CONVERSION
446C
4WHSV
457C
468C
4WHSV
421
WHSV

FIG. 5

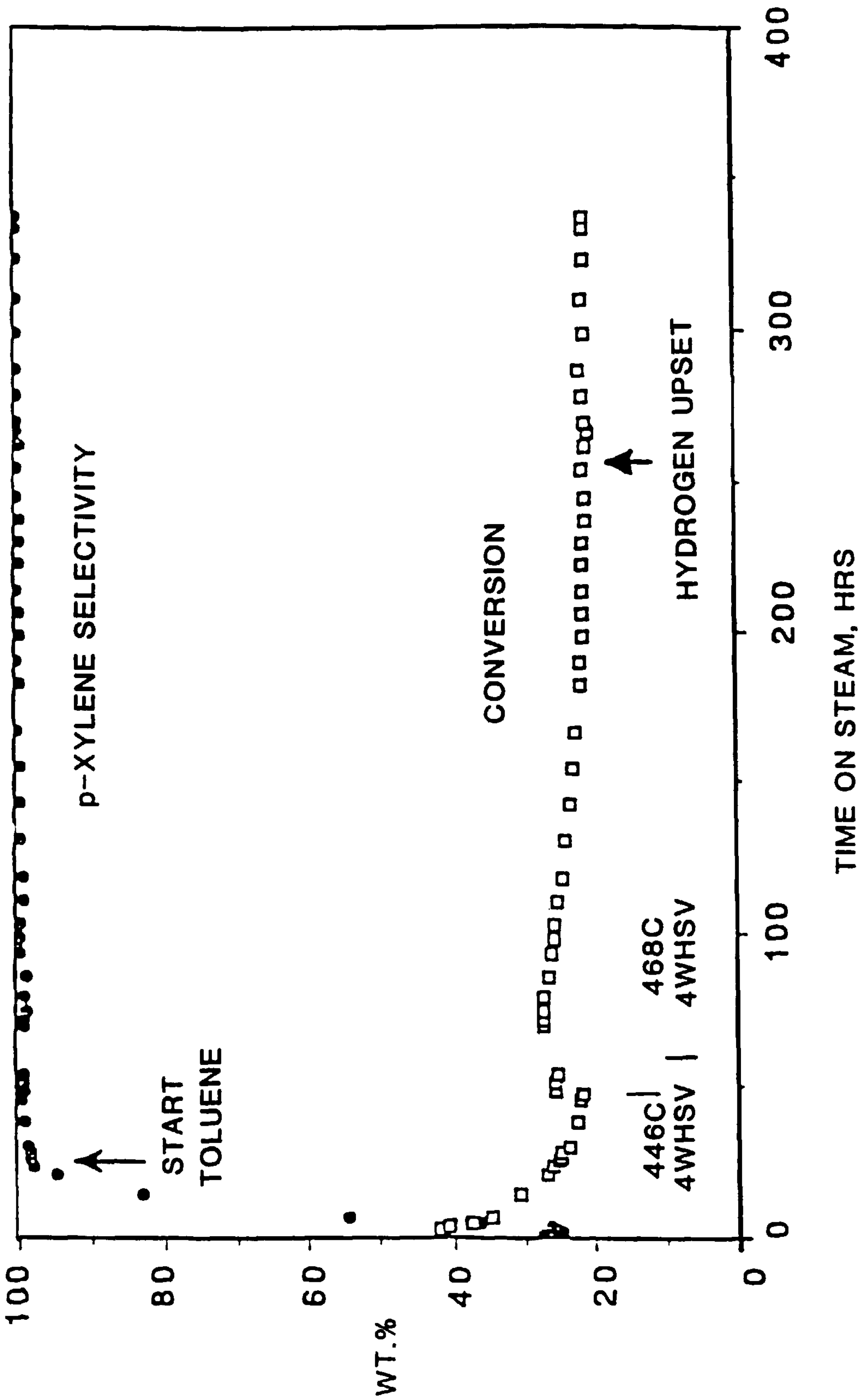
p-XYLENE SELECTIVITY
CONVERSION
HYDROGEN UPSET
START
TOLUENE
446C
4WHSV
468C
4WHSV
WT.%
100
80
60
40
20
0
0
100
200
300
400
TIME ON STEAM, HRS

FIG. 6